# EUROPEAN PATENT APPLICATION

(11) **EP 4 722 233 A1**
(43) Date of publication of application: **08.04.2026**
(21) Application number: 24383063.5
(22) Date of filing: 02.10.2024
(51) Int. Cl.: C07K 14/415, C12N 15/82

(54) **METHOD TO IMPROVE PLANT GERMINATION AND FRUIT QUALITY UNDER STRESS CONDITIONS**

(71) Applicant: Universidad Politécnica De Madrid, 28040 Madrid (ES); Universidad Politécnica De Valencia (UPV), 46022 Valencia (ES); Consejo Superior De Investigaciones Científicas - CSIC, 28006 Madrid (ES)
(72) Inventor: CASTELLANO MORENO, María del Mar, 28006 Madrid (ES); GÓMEZ MENA, María Concepción, 46022 Valencia (ES); MANGANO, Silvia, 28040 Madrid (ES); MUÑOZ GUTIÉRREZ, Alfonso, 28040 Madrid (ES); TORIBIO LÓPEZ, Francisco René, 28040 Madrid (ES)
(74) Representative: Elzaburu S.L.P.

(57) **Abstract**

The present invention relates to a genetic construct that allows overexpression in plants of the Arabidopsis HOP3 protein (AtHOP3) under the specific Arabidopsis BIP3 promoter (AtBIP3 promoter or pAtBIP3) under high salt conditions. The present invention also relates to transgenic plants, or parts thereof, comprising the genetic construct of the present invention, and to the use of these plants in crops subjected to environmental stress or any other condition that courses with ER stress.

## Description

### FIELD OF THE INVENTION

The present invention relates to the field of plants subjected to stress conditions, particularly to high salt conditions, and to provision of a method to improve plant tolerance, seed germination, seedling establishment and fruit quality and ripening under these stress conditions.

### BACKGROUND OF THE INVENTION

Soil salinity is one of the most devastating environmental stresses in agro-economic terms, since this stress limits the surface of land that can be cultivated and causes significant losses in the productivity and quality of crops (Shrivastava and Kumar., 2015, Saudi J Biol Sci. 22(2):123-31). It has been estimated that 20% of total cultivated land and 33% of irrigated agricultural land worldwide are currently affected by high salinity. Furthermore, it has been calculated that each year the surface of the Earth affected by this stress increases by 10%. This increase is largely due to the decrease in precipitation and the increase in surface evaporation associated with climate change, irrigation with saline water and excessive fertilization. For this reason, it is estimated that more than 50% of arable land will be considered saline in the year 2050 (Jamil et al., 2011, Crit. Rev. Plant Sci. 30(5):435-458). This fact is especially relevant considering that high salt limits production in all major crops. In fact, some of the agricultural crops most sensitive to soil salinity are corn, strawberries, peas, beans (whose yield decreases even with the slightest salinity). Other crops whose production is affected by the moderate increase of soil salinity are rapeseed, barley, rye, oats or tomatoes. All this is currently causing million-dollar annual losses in the production of these crops. Furthermore, as a consequence of the increase in the surface area affected by salinity, these losses are expected to increase and cause significant food security problems (Godfray et al., 2010, Science 327:812-818; IPCC 2014, Climate Change 2014:Mitigation of Climate Change. Contribution of Working Group III to the Fifth Assessment. Report of the Intergovernmental Panel on Climate Change. Edenhofer, O., R. Pichs-Madruga, Y. Sokona, E. Farahani, S. Kadner, K. Seyboth, A. Adler, I. Baum, S. Brunner, P. Eickemeier, B. Kriemann, J. Savolainen, S. Schlömer, C. von Stechow, T. Zwickel and J.C. Minx (eds.). Cambridge University Press, Cambridge, United Kingdom and New York, NY, USA.).

One of the main negative effects of the soil salinization process is the alteration of water absorption by plants (also called osmotic stress). In addition to osmotic stress, high salinity also produces ionic stress in plants. This stress occurs due to the increase in the concentration of toxic ions, such as chloride (CI-) or sodium (Na+) and the decrease in ions essential for crop development (such as K+). Soil salinity also reduces the assimilation of nutrients in plants, which causes a decrease in their growth and affects the chlorophyll concentration and the photosynthetic rate. Due to its negative impact on plant physiology, soil salinity produces a significant reduction in crop growth and production even in moderately saline soils. These effects completely inhibit the general growth of plants under conditions of severe salt stress Shrivastava and Kumar., 2015, Saudi J Biol Sci. 22(2):123-31; Pardo., 2010, Curr Opin Biotechnol. 21(2):185-96.).

Given this situation, in order to alleviate production losses associated with environmental stresses (such as salt stress), the Food and Agriculture Organization of the United Nations (https://www.fao.org/home/es) proposes significantly improving agricultural management techniques and using varieties that are more tolerant to different stresses.

The reasons given above highlight the need to develop new biotechnological tools and strategies that overcome the problem of improving plant tolerance and fruit quality in fields and soils affected by high salt concentrations.

Arabidopsis HOP3 (AtHOP3) is a protein belonging to the HSP70-HSP90 organizing protein co-chaperone family. This protein is involved in signaling of hormonal pathways, such as jasmonic acid, auxins and gibberellins (Muñoz et al., 2021, Plant Physiology 187(3), 10; Muñoz et al., 2022, Plant Cell Environ 45, 2508-2519; Mangano et al., 2023, Plant Commun, 100517). Since these hormonal networks control multiple aspects of plant development such as root development, growth, etc. the ectopic expression of AtHOP3 is believed to cause serious problems in development in plants.

AtBIP3 promoter (pAtBIP3) refers to the Arabidopsis BIP3 promoter, an inducible promoter which is active under conditions of ER (endoplasmic reticulum) stress. This stress happens in response to adverse environmental conditions, such as high salinity, drought or in response to pathogen attack (Martinez and Chrispeels, 2003, Plant Cell, 15, 561-76).

### SUMMARY OF THE INVENTION

The present invention overcomes the problems of the state of the art by showing that plants that ectopically express the Arabidopsis HOP3 protein (AtHOP3) under the specific AtBIP3 promoter (pAtBIP3) exhibit greater tolerance and a substantial improvement in fruit quality under high salt conditions.

A first aspect of the present invention relates to a recombinant DNA molecule pAtBIP3:AtHOP3, comprising the DNA sequence of the AtBIP3 promoter (pAtBIP3) (SEQ ID NO 1) and the DNA sequence coding for the Arabidopsis HOP3 protein, AtHOP3 (SEQ ID NO 2), wherein expression of protein AtHOP3 is under the control of the AtBiP3 promoter.

The recombinant DNA molecule, when transformed in plants, makes possible that expression of AtHOP3 is under the control of the AtBiP3 promoter, which allows the selective induction of the expression of AtHOP3 under ER stress conditions.

A second aspect of the present invention relates to a method of obtention of the recombinant DNA molecule of above.

A third aspect of the present invention relates to the use of AtHOP3 expression under the AtBiP3 promoter to enhance seed germination and seedling establishment rate and improve fruit quality and ripening in plants under stress conditions, particularly under high salt conditions.

A fourth aspect of the present invention relates to transgenic plants, or part thereof, comprising the genetic construct pAtBIP3:AtHOP3.

Further aspects of the present invention correspond to a method of producing a transgenic plant, or part thereof, comprising the genetic construct pAtBIP3:AtHOP3. Preferably, the method comprises transforming plants with the genetic construct pAtBIP3:AtHOP3 to produce transformed plants.

Yet another aspect corresponds to the use of the transgenic plants or part thereof, in crops subjected to environmental stress conditions.

The unique combination of AtHOP3 overexpression under the AtBiP3 promoter, which is specifically inducible under ER stress conditions, allows overexpression of the AtHOP3 protein in young and adult plants specifically under ER stress conditions. Basically, in plants expressing the pAtBIP3:AtHOP3 construct, the ectopic AtHOP3 protein is only accumulated when the plant is subjected to ER stress, but not in the absence of such stress. Under control conditions, the plant does not accumulate ectopic AtHOP3, so it behaves like a wild-type plant. Differently, under salt stress conditions, ER stress occurs in the plant, activating the AtBIP3 promoter and allowing the ectopic accumulation of the AtHOP3 protein. As described in the present invention, the ectopic expression of AtHOP3 under the AtBIP3 promoter allows plants to reduce the damage caused by salt stress. As a consequence, the plants grow and adapt better to the presence of high salt concentrations, and the fruit quality and ripening are improved. In addition, the increase in tolerance to high salt concentrations is carried out without influencing the normal development of plants in the absence of stress. These effects represent a high advantage in agricultural terms.

Thus, expression of the AtHOP3 protein under the AtBIP3 promoter can be used as a biotechnological tool to significantly improve the tolerance of plants to salt stress and, therefore, the yield and sustainability of crops in fields affected by salinity. The fact that the combination of AtBIP3 promoter and AtHOP3 in the pAtBIP3:AtHOP3 DNA molecule allows the AtHOP3 protein to be ectopically expressed selectively under conditions of ER stress, which occurs in conditions of salt stress, but also in response to infection by pathogens, heat or drought, makes it highly possible that this approach can efficiently improve crop yields and their sustainability in fields subjected to high temperatures and drought, in crops affected by pests and pathogens or in crops subjected to other stresses or conditions that course with ER stress or a combination of them.

Inducing the AtHOP3 expression by the AtBIP3 promoter has unexpectedly proven to be an excellent means to increase plant tolerance and fruit quality under salt stress conditions, without influencing normal plant development in the absence of stress.

### BRIEF DESCRIPTION OF FIGURES

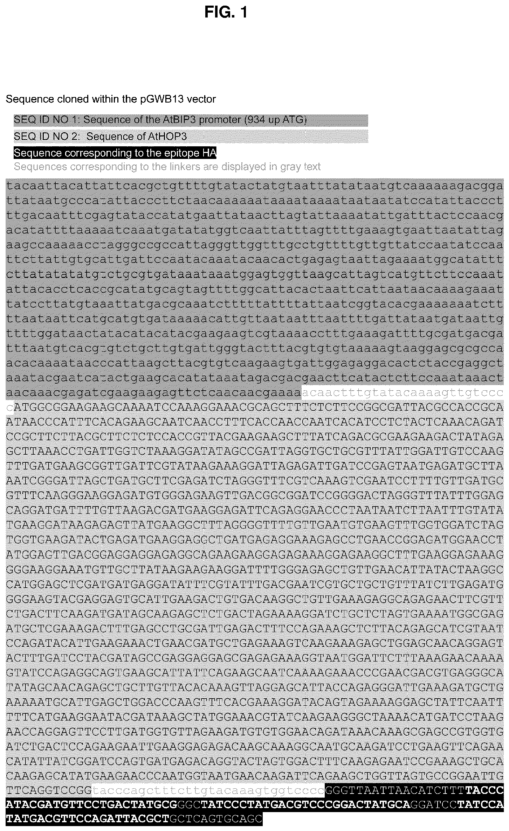

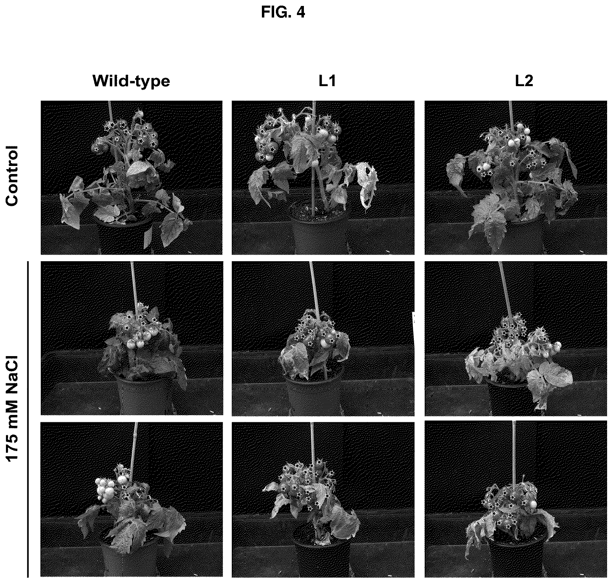

**Figure 1****:**
   Sequence cloned within the pGWB13 vector.
   The sequence of the AtBIP3 promoter (SEQ ID Nº 1) is displayed in lower case and highlighted in dark grey.
   The DNA sequence coding for the Arabidopsis HOP3 protein (SEQ ID Nº 2), AtHOP3, is displayed in upper case and highlighted in light grey.
   The sequences corresponding to the linkers that were used for cloning are displayed in grey letter.
   The sequence corresponding to the epitope HA (hemagglutinin) is displayed in upper case, white letter and highlighted in black. The -HA is only an epitope that allows the protein to be recognized by western-blot but does not interfere in its function).
   The whole sequence displayed in Fig. 1 constitutes SEQ ID Nº 3.
**Figure 2****:**
   Analysis of AtHOP3 expression by RT-qPCR (A) and AtHOP3 ectopic protein accumulation by western-blot using the anti-HA antibody (B) in the wild type genotype (Col-0) and in three different pAtBIP3:AtHOP3 lines (*pBIP3-1, pBIP3-2* and *pBIP3-3*) of *Arabidopsis thaliana* in the absence or presence of the ER stress inducer 1,4-Dithiothreitol (DTT). In (A), the AtHOP3 expression was relative to the expression of a calibrator gene, in this case the PP2A gene. Samples that present significant differences (analyzed using a one-way ANOVA test) have been marked with asterisks (**, p value ≤ 0.01; ***, p value ≤ 0.001).
**Figure 3****:**
   Salinity tolerance analysis of Col-0 plants (wild-type plants) and pAtBIP3:AtHOP3 transgenic lines of *Arabidopsis thaliana.* (A) Representative images of 7-day-old seedling of the different lines grown in the absence (control, top panel) or presence of 150 mM NaCl (lower panel). (B) Quantification of the percentage of seedlings with the presence of fully expanded green cotyledons after growth for 7 days in high salt. The percentage of fully expanded green cotyledons in control conditions was 100% in all lines. Values represent the mean ±SEM of three independent replicates. The samples that present significant differences with respect to Col-0 (analyzed using a univariate ANOVA test) have been marked with asterisks (***, p value ≤ 0.001).
**Figure 4****:**
   Salinity tolerance analysis of the wild-type line and different pAtBIP3:AtHOP3 transgenic tomato lines (L1 and L2). Representative images of plant growth of the different lines in the absence (control, upper panel) or presence (lower panels) of 175 mM NaCl. After 21 days of growth in soil, the plants began to be watered every 3 days for 21 days with water containing or not 175 mM NaCl. Subsequently, irrigation was continued every 3 days with water until the fruits reached maturity. The plants were photographed and the fruits were collected for further analysis. Red (ripen) tomatoes are marked with an asterisk.
**Figure 5****:**
   Analysis of fruit quality of tomato plants from the wild-type line and different tomato pAtBIP3:AtHOP3 transgenic lines (L1 and L2) under control and high salinity conditions. (A) Representative images of fruits obtained from wild-type plants and from the transgenic lines L1 and L2 grown in the absence of salt stress (control, upper panel) or watered in the high salinity regime described in Figure 4 (lower panels). (B) Quantification of the percentage of red (ripen) fruits over the total fruits obtained per plant. (C) Quantification of the content of total soluble sugars in the fruit measured as brix degrees (Brixº). (D) Quantification of the caliber of the fruits obtained by each plant. In B-D, the samples that present significant differences with respect to the wild-type line (analyzed by a univariate ANOVA test) have been marked with asterisks (*, p value ≤ 0.05; **, p value ≤ 0.01; ***, p value ≤ 0.001). Values represent the mean ±SEM of 6 plants. Each experiment was repeated 3 times obtaining similar results.
**Figure 6****:**
   Analysis of the physiological status of tomato plants from the wild-type and the L1 and L2 transgenic lines under control and salinity conditions. Analysis of different parameters in leaves. Plants, wild-type and pAtBIP3:AtHOP3, were grown for 21 days in soil. After this period, they began to be watered every 3 days for 21 days with water (control) or with a solution containing 175 mM NaCl. At that time, leaf 3 was taken and analyzed. Panels correspond to quantification of (A) Na+ concentration, (B) K+ concentration, (C) percentage of ion loss (or ion leakage) as a measure of cellular damage, (D) total protein content, (E) chlorophyll A (Chla), (F) total chlorophyll (Total chlorophyll). Values represent the mean ±SEM of 6 plants of a representative experiment. The experiment was repeated 3 times obtaining similar results. The samples that present significant differences with respect to the wild-type line (analyzed by a univariate ANOVA test) have been marked with asterisks (*, p value ≤ 0.05; **, p value ≤ 0.01; ***, p value ≤ 0.001).

### DESCRIPTION OF THE INVENTION

A first aspect of the present invention relates to a recombinant DNA molecule comprising the DNA sequence of the specific AtBIP3 promoter, pAtBIP3 (SEQ ID NO: 1) and the DNA sequence coding for the Arabidopsis HOP3 protein, AtHOP3 (SEQ ID NO: 2), wherein expression of protein AtHOP3 is under the control of the AtBiP3 promoter, pAtBIP3.
SEQ ID NO: 1: DNA Sequence of the specific AtBIP3 promoter (pAtBIP3).
SEQ ID NO: 2: DNA sequence coding for the gene HOP3 of Arabidopsis thaliana (AtHOP3).

The genetic construction comprising the sequence of AtHOP3 gene, operably linked to the sequence of the AtBIP3 promoter (pAtBIP3), as well as genetic constructions comprising sequences with significant sequence identity of to one or both corresponding sequences, are named pAtBIP3:AtHOP3, herein.

For significant sequence identity it is understood at least 95 percent sequence identity, at least 96 percent sequence identity, at least 97 percent sequence identity, at least 98 percent sequence identity, at least 99 percent sequence identity, and at least 100% identity.

As used herein, the term "sequence identity" refers to the extent to which two optimally aligned polynucleotide sequences or two optimally aligned polypeptide sequences are identical.

The method of obtention of the recombinant DNA molecule of claim 1 of the present invention is any possible molecular biology method available in the state of the art for provision of recombinant DNA molecules.

Transgenic plants comprising the genetic construct pAtBIP3:AtHOP3 display expression of AtHOP3 under the AtBiP3 promoter.

In addition to transgenic plants comprising the genetic construct pAtBIP3:AtHOP3, the present invention relates to parts of these plants comprising the recombinant DNA molecule. Preferably, as part of a plant is meant any possible part of the plant, including cells and seeds.

In particular embodiments, a progeny plant of the transgenic plant, or part thereof or a transgenic cell or seed comprising the recombinant DNA molecule are also provided by the invention.

The transgenic plants of the present invention might be monocotyledonous and dicotyledonous plants.

In one embodiment, the monocotyledonous plant is selected from the group consisting of Maize (Zea mays), Rice (Oryza sativa), Wheat (Triticum), Barley (Hordeum vulgare), Sorghum (Sorghum spp.), Millet, Pearl Millet (Pennisetum glaucum), Finger Millet (Eleusine coracana), Preso Millet (Panicum miliaeeurn), Foxtail Millet (Setaria italica), Oats (Avena sativa), Triticale, Rye (Secale cereale), Fonio (Digitaria), Onions (Alliutn spp.), Pineapple (Allanas spp.), Turfgrass, Sugarcane (Saccharum spp.), Palm (Arecaceae), Bamboo (Bambuseae), Banana (Musaceae), Ginger family (Zingiberaceae), Lilies Daffodils

(Narcissus), Irises (Iris), Amaryllis, Orchids (Orchidaceae), Cannas, Bluebells (Hyacinthoides), and Tulips (Tulipa).

The transgenic plant may also be a dicotyledonous plant. In one embodiment, the dicotyledonous plant is selected from the group consisting of Soybean (Glycine max), Wild Soybean (Glycine soja), Cotton (Gossypium), Tomato (Solanum lycopersicum), Pepper (Piper), Squash (Cucurbita), Pea (Pisum sativum), Alfalfa (Medicago sativa), Medicago truncatula, Beans (Phaseolus), Chick pea (Cicer arietinum), Sunflower (Helianthus annuus), Patato (Solanum tuberosum), Peanut (Arachis hypogaea), Quina, Buckwheat (Fagopyrum esculentum), Carob (onia siliqua), Beet (Beta vulgaris), Spinach (Spinacia oleracea), and Cucumber (Cucumis sativos).

Most preferably, the transgenic plant, or part thereof, belong to the *Arabidopsis thaliana sp.* or is *Solanum lycopersicum.*

Any possible method known in the state of the art for transforming a plant with the genetic construct pAtBIP3:AtHOP3 might be employed for obtaining the transgenic plant, or part thereof, of the present invention. Thus, the method of producing a transgenic plant, or part thereof, comprising the genetic construct pAtBIP3:AtHOP3, might be any method known in the art for the skilled person.

In a preferred embodiment of the present invention, the method of producing transgenic plants or part thereof comprises transforming flowers or cotyledon explants with the genetic construct pAtBIP3:AtHOP3 to produce transformed plants.

Preferably, the method used for Arabidopsis transformation is the floral dip method. This method generates transgenic seeds that further generate transgenic plants directly.

Preferably, the method used for tomato transformation consists on transformation of cotyledon explants. In this case, transformed cells form callus that are used to regenerate whole transgenic plants. With the transformation methods used, several independent transgenic lines are generated that contain the construction in different parts of the genome.

Some of the effects displayed by the transgenic plants of the present invention include improvement of seed germination and seedling establishment rate, as well as improvement of fruit quality and ripening in plants subjected to stress conditions.

For stress conditions in the present invention it is understood high salt conditions, as well as high temperatures, drought, and crops affected by pests and pathogens or any other condition that courses with ER stress or a combination of them. Most preferably, the stress conditions of the present invention relate to high salt conditions.

In the results of the present invention, it is demonstrated that the ectopic expression of the AtHOP3 protein under the AtBIP3 promoter improves tolerance to high salinity stress in different types of plants, such as Arabidopsis and tomato. As displayed in the Examples below, and in particular as deduced from the DTT experiments, the pAtBIP3 is induced in response to ER stress, and thereby, it will in principle induce and promote AtHOP3 expression in any situation that courses with ER stress.

Expression of the AtHOP3 protein under the AtBIP3 promoter, under salt stress conditions, increases chlorophyll concentration and improves ion balance, decreases damage caused by high salt concentrations and improves photosynthetic capacity and total protein production. In addition, the expression of AtHOP3 under the AtBIP3 promoter promotes fruit ripening and improves fruit quality under salt stress conditions. All this is achieved without significantly interfering with plant growth in the absence of stress.

These results demonstrate that the expression of the AtHOP3 protein under the AtBIP3 promoter can be used as a biotechnological tool to significantly improve the tolerance of plants to salt stress and, therefore, the yield and sustainability of crops in soils affected by salinity.

The term "comprising" or "comprise" or "comprises" includes the term "consisting of" or "consist of" or "consists of".

Several preferred embodiments of the present invention will be described in greater detail below by means of specific examples.

### EXAMPLES

The following examples illustrate the present invention and are not to be understood as limiting the invention. In particular, the examples displayed correspond to

### Example 1.

Cloning of "pAtBIP3:AtHOP3" construct. The construct obtained is displayed in Figure 1. The construct pAtBIP3:AtHOP3 was obtained by cloning the AtBIP3 promoter and the AtHOP3 coding sequence in frame with the HA epitope by recombination of the corresponding pDONR plasmids (pDONR221P1P5R and pDONR221P5P2P, respectively (Gateway cloning Invitrogen, Karlsruhe, Germany)) with pGWB13 (Nakagawa *et al.,* 2007).

### Example 2.

The pAtBIP3:AtHOP3 construct was introduced in Arabidopsis by the floral dip method (Clough SJ, Bent AF.1998.Plant J.16(6):735-43), corresponding transgenic plants being successfully obtained. Plants with a single insertion were selected. Among all the lines obtained, three independent homozygous lines were selected, which were designated with the name:
pAtBIP3:AtHOP3 line #1: pBIP3-1.
pAtBIP3:AtHOP3 line #2: pBIP3-2.
pAtBIP3:AtHOP3 line #3: pBIP3-3.

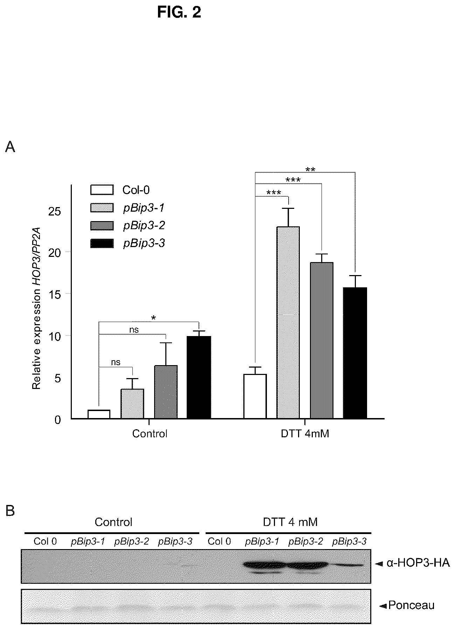

To verify that the correct expression of the transgene in these lines is induced in response to ER stress, the expression of the AtHOP3 transgene in response to 4 mM of 1-4 Dithiothreitol (DTT) an agent that induces ER stress, was evaluated by qRT-PCR. As it can be seen in Figure 2, AtHOP3 expression is induced in the *pBIP3-1, pBIP3-2* and *pBIP3-3* lines in the presence of DTT. Furthermore, it was verified by western-blot that the ectopic AtHOP3 protein did not accumulate under control conditions and only accumulates in the presence of ER stress (induced in this case with DTT).

### Example 3.

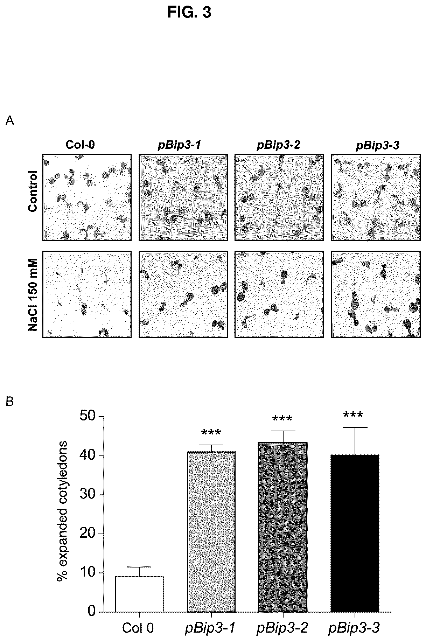

Once the correct expression of the transgene and the correct accumulation of AtHOP3 under the AtBIP3 promoter in the different pAtBIP3:AtHOP3 independent lines had been demonstrated, it was evaluated whether these lines could show an enhanced performance under high salt conditions. As displayed in Figure 3, compared to the wild-type genotype (Col-0), plants that ectopically express AtHOP3 under the AtBIP3 promoter show better development in the presence of high salinity, presenting a higher percentage of plants with green and expanded cotyledons.

### Example 4.

Once the proof of concept was carried out in Arabidopsis, in order to test whether the ectopic expression of AtHOP3 under the AtBIP3 promoter could also improve the tolerance to salt stress of species of agronomic interest, transgenic tomato plants (Microtom variety) that expressed the pAtBIP3:AtHOP3 construct were generated by cotyledon transformation, following the procedure described in Ellul et al. 2003. Theoretical and Applied Genetics. 106: 231-238. Two independent lines were selected, carried out to homogeneity and analyzed. In this case these lines were called:
pAtBIP3:AtHOP3-line #1: L1
pAtBIP3:AtHOP3line #2: L2

To study whether the expression of AtHOP3 under the AtBIP3 promoter could improve tolerance to high salinity in tomato plants, wild-type and transgenic L1 and L2 plants were irrigated with water (control condition) or with a 175 mM NaCl solution.

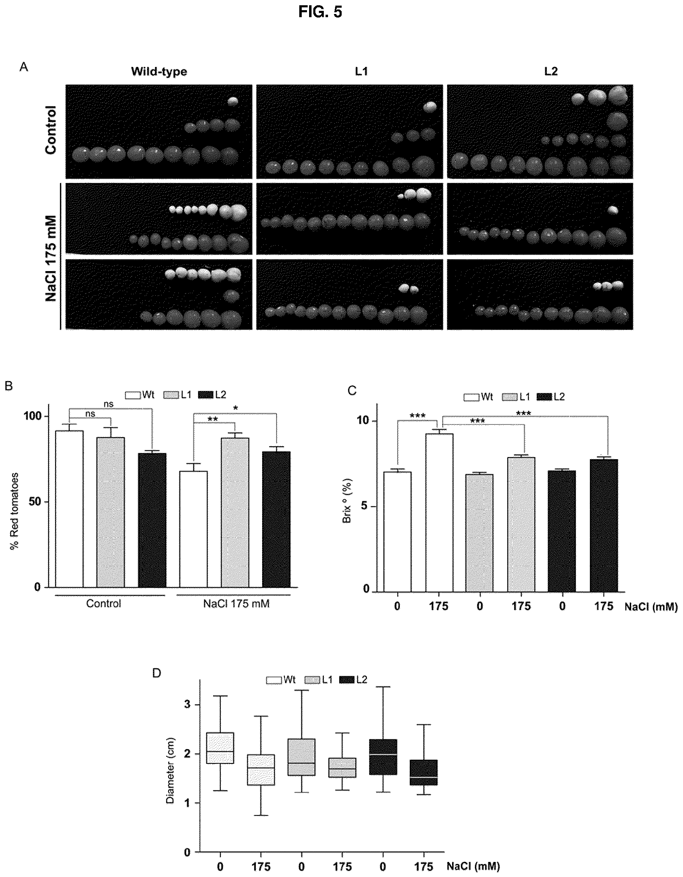

As seen in Figures 4 and 5 A-B, there were no significant differences in control conditions regarding the production of red fruits, brix degrees (Figure 5 C) or fruit caliber (Figure 5 D) between wild-type plants and L1 and L2 plants. This denotes that the development of these plants in the absence of stress is similar to that of wild-type plants. However, tomato plants that express AtHOP3 under the AtBIP3 promoter (L1 and L2 lines) have a greater number of red fruits than wild-type plants when subjected to high salinity, resulting in a significant increase in yield (Figure 4, 5 A-B). Furthermore, as a consequence of salt stress, the fruits of wild-type tomato plants increase their total content of soluble sugars, a conclusion that comes from the increase observed in Brix degrees (Figure 5 C). However, the fruits of plants that express AtHOP3 under the AtBIP3 promoter maintain the total concentration of soluble sugars (Brix degrees) under salt conditions similar to tomatoes that have not undergone stress (Figure 5 C). These data demonstrate that, under stress conditions, plants that express AtHOP3 under the AtBIP3 promoter have a higher fruit quality than wild-type plants and that this quality is similar to that of tomatoes which have not undergone salt stress.

### Example 5.

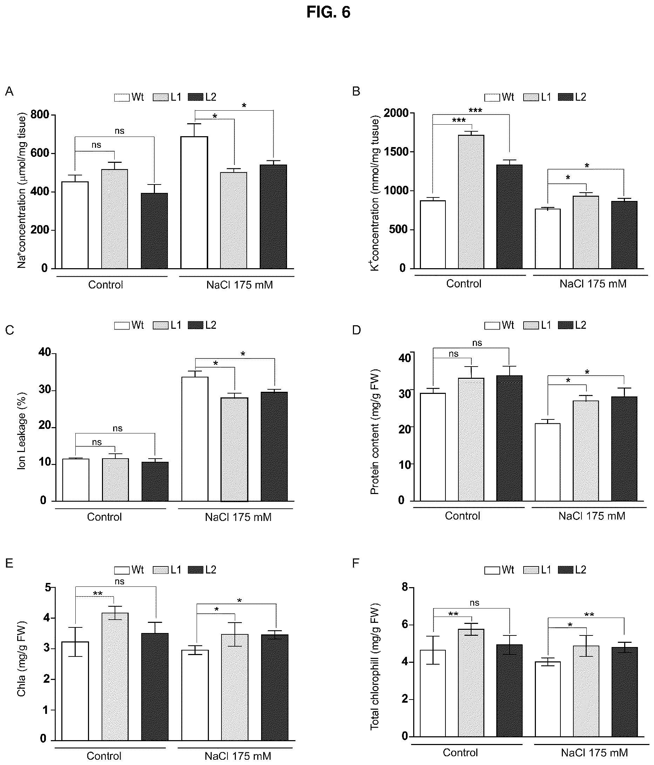

Various parameters were analysed in the tomato plants of the precedent examples, to assess their physiological status both in control conditions and in the presence of high salt. As shown in Figure 6, tomato plants expressing AtHOP3 under the AtBIP3 promoter behave broadly similar to wild-type plants under control conditions. In some cases, some of the lines showed significant differences in certain parameters under control conditions that are not found in both lines, such as the increase in chlorophyll A and total chlorophyll in line L1. An increase in the K+ concentration is also observed in the case of lines L1 and L2. In all cases the variations in these parameters are associated with an improvement in the physiological status of such plants.

Under salt stress conditions, compared to the wild-type genotype, plants that express AtHOP3 under the AtBIP3 promoter consistently and significantly show:
- Lower sodium and higher potassium contents (Figure 6 B), which implies a better ionic balance.
- Less damage to leaves (expressed as ion loss or ion leakage) (Figure 6 C).
- A greater amount of chlorophyll A and total chlorophyll, which denotes greater photosynthetic efficiency (Figure 6 E-F).
- Greater amount of total protein, which, together with the increase in chlorophyll, denotes better assimilation of nutrients (Figure 6 D)

All these physiological data demonstrate that plants that express AtHOP3 under the AtBIP3 promoter are significantly more tolerant to salt stress, which allows the generation of fruits of higher quality and maturity. This tolerance is acquired without presenting negative physiological or developmental effects in the absence of stress.

These experiments demonstrate that the expression of AtHOP3 under the AtBIP3 promoter is a biotechnological tool that allows increasing the tolerance of plants to salt stress. The pAtBIP3:AtHOP3 combination allows the AtHOP3 transgene to be expressed selectively under conditions of ER stress. This stress occurs under conditions of salt stress, but also in response to infection by pathogens, heat, drought, etc. Therefore, it is highly probable that this strategy allows increasing the tolerance of plants, not only to salt stress, but also to these other kinds of stresses or conditions that course with the induction of ER stress or a combination of them.

## Claims

1. A recombinant DNA molecule comprising the DNA sequence of the AtBIP3 promoter (pAtBIP3) (SEQ ID NO: 1) and the DNA sequence coding for the Arabidopsis HOP3 protein, AtHOP3 (SEQ ID NO: 2), wherein expression of protein AtHOP3 is under the control of the AtBiP3 promoter.

2. Method of obtention of the recombinant DNA molecule of claim 1.

3. Use of AtHOP3 expression under the AtBiP3 promoter to promote seed germination and seedling establishment rate and improve fruit quality and ripening in plants under stress conditions.

4. The use of claim 3 wherein the stress conditions consist in high salt conditions.

5. A transgenic plant, or part thereof, comprising the genetic construct pAtBIP3:AtHOP3.

6. The transgenic plant, or part thereof, of claim 5 wherein said transgenic plant is a monocotyledonous plant or a dicotyledonous plant.

7. The transgenic plant, or part thereof, of claim 6 wherein the plant belongs to the *Arabidopsis thaliana sp.* or is *Solanum lycopersicum.*

8. Method of producing the transgenic plant, or part thereof, of claims 5 to 7, by transforming plants with the genetic construct pAtBIP3:AtHOP3.

9. The method of claim 8 wherein the transgenic plan is *Arabidopsis thaliana sp* and the transformation method is the floral dip method, wherein transgenic seeds are generated that further generate transgenic plants directly.

10. The method of claim 8 wherein the transgenic plan is *Solanum lycopersicum* and the transformation method consists on transformation of cotyledon explants, wherein transformed cells form callus are used to regenerate whole transgenic plants.

11. Use of the transgenic plant, or part thereof, of claims 5 to 7, in crops subjected to environmental stress conditions or any other condition that courses with ER stress or a combination of them.

12. The use of claim 11 wherein the environmental stress conditions are selected from high salt stress conditions, pest and pathogen infections, high temperatures and drought.
